# EUROPÄISCHE PATENTSCHRIFT

(11) **EP 1 453 774 B1**
(45) Veröffentlichungstag und Bekanntmachung des Hinweises auf die Patenterteilung: **21.01.2009**
(21) Anmeldenummer: 02804186.1
(22) Anmeldetag: 20.11.2002
(51) Int. Cl.: C07B 37/04

(54) **VERFAHREN ZUR ARYLIERUNG VON OLEFINEN**
METHOD FOR THE ARYLATION OF OLEFINS
PROCEDE D'ARYLATION D'OLEFINES

(30) Priorität: 03.12.2001 DE 10159270
(43) Veröffentlichungstag der Anmeldung: 08.09.2004
(73) Patentinhaber: Saltigo GmbH, 40764 Langenfeld (DE)
(72) Erfinder: RAMPF, Florian, 50733 Köln (DE); ECKERT, Markus, 200050 Shanghai (CH)
(74) Vertreter: Wichmann, Birgid
(86) Internationale Anmeldenummer: PCT/EP2002/012993
(87) Internationale Veröffentlichungsnummer: WO 2003/048079

(56) Entgegenhaltungen:
- CHRISTOPH GÜRTLER AND STEPHEN L. BUCHWALD: "A Phosphane Free Catalyst System for the HEck Arylation of Disubstituted Alkenes: Application to the Synthesis of Trisubstituted Olefins" CHEM. EUR. J., Bd. 5, Nr. 11, 1999, Seiten 3107-12, XP002244142
- SABINE BERTEINA, SEBASTIAN WENDEBORN, WOFGANG K.-D. BRILL AND ALAIN DE MESMAEKER: "Pd mediated C-C Bond Formation with Olefins and Acetylenes on SOlid Support: A Scope and Limitations Study" SYNLETT, Bd. 6, 1998, Seiten 676-8, XP002244143
- ADAM F. LITTKE AND GREGORY C. FU: "A versatile catalyst for Heck reactions of aryl chlorides and aryl bromides under mild conditions" JOURNAL OF THE AMERICAN CHEMICAL SOCIETY, AMERICAN CHEMICAL SOCIETY, WASHINGTON, DC, US, Bd. 123, Nr. 29, 2001, Seiten 6989-7000, XP002236859 ISSN: 0002-7863 in der Anmeldung erwähnt
- BELETSKAYA IRINA P ET AL: "The Heck Reaction as a Sharpening Stone of Palladium Catalysis" CHEMICAL REVIEWS, AMERICAN CHEMICAL SOCIETY. EASTON, US, Bd. 100, Nr. 8, 2000, Seiten 3009-3066, XP002199991 ISSN: 0009-2665

## Beschreibung

Die Erfindung betrifft ein Verfahren zur Arylierung von Olefinen durch Umsetzung von Halogenaromaten oder Arylsulfonaten mit Olefinen in Gegenwart eines Palladium-katalysators, einer sterisch anspruchsvollen Stickstoffbase und in Gegenwart eines Salzes.

Viele Arylolefine besitzen als Feinchemikalien, UV-Absorber, Ausgangsprodukte für Polymere und Wirkstoffzwischenprodukte eine hohe industrielle Bedeutung.

Die Darstellung von Arylolefinen erfolgt häufig durch Palladium-katalysierte Kupplung von Iod- oder Bromaromaten, seltener Chloraromaten oder Arylsulfonaten, mit Olefinen. Aufgrund des hohen Preises und der durch die hohe Molmassen bedingten großen Abfallmengen ist der Einsatz von Iod- und Bromaromaten in technischem Maßstab nachteilig. Die leichter verfügbaren und daher günstigeren Chloraromaten zeigen jedoch vergleichsweise geringe Reaktivität.

Zapf und Beller (Chem. Eur. J. 2001, 7, 2908) beschreiben unter Anderem die Palladium-katalysierte Umsetzung von Chloraromaten mit Olefinen bei Temperaturen von 160°C und Zusatz von 20 mol-% von quartemären Ammoniumsalzen in Gegenwart einer Base. Dabei werden in 24 h Katalysatorumsatzzahlen (TON) von 850-1000 erreicht.

Nachteilig ist bei diesem Verfahren jedoch der hohe Bedarf an quartemären Ammoniumsalzen und die geringen Katalysatorumsatzfrequenzen (TOF) von max. 42 pro Stunde.

Littke und Fu (J. Am. Chem. Soc. 2001, 123, 6989) beschreiben ein Verfahren, in dem Chloraromaten mit Olefinen bei Raumtemperatur unter Verwendung von Palladiumdibenzylidenaceton ([Pd₂(dba)₃]) und Tri-tert-butylphosphin in Gegenwart von Dicyclohexylmethylamin umgesetzt werden. Allerdings sind für das beschriebene Verfahren große Mengen an Palladiumkatalysator nötig, was die technische Anwendung unwirtschaftlich macht.

Gürtler und Buchwald (Chem. Eur. J., Bd. 5, 1999, 3107-3112) offenbaren ein Verfahren zur Olefinierung von Arylbromiden und -iodiden (Heckreaktion), katalysiert durch Palladium ohne Phosphine in Gegenwart von ein sterisch anspruchsvollen Stickstoffbase, Tetraethylammoniumchlorid und Dimethylacetatmid.

Es bestand daher das Bedürfnis ein Verfahren zu entwickeln, das die Kupplung von Halogenaromaten, insbesondere Chloraromaten mit Olefinen in effizienter Weise ermöglicht.

Es wurde nun ein Verfahren zur Herstellung von Arylolefinen gefunden, das dadurch gekennzeichnet ist, dass
- aromatische Verbindungen der allgemeinen Formel (I),

   Ar-[X]ₙ (I),

   in der
   - n: für eins oder zwei steht und
   - Ar: für einen substituierten oder unsubstituierten aromatischen Rest und
   - X: jeweils unabhängig für Chlor, Brom, Iod oder ein Sulfonat steht
- in Gegenwart eines Palladium-Katalysators der allgemeinen Formel (IV) werden,

   [PdL₂An₂] (IV)

   in der
   - L: für jeweils eine Monophosphorverbindung oder
   - L₂: zusammen für ein Diphosphorverbindung und
   - An: für ein Anion, bevorzugt für Chlorid, Bromid, Iodid, Acetat, Propionat, Allyl oder Cyclopentadienyl steht.
   oder solche der allgemeinen Formel (IVb)

   [PdLₙ] (IVb)

   in der n für 2, 3 oder 4 und
   in der
   - L: jeweils für eine Monophosphorverbindung oder ein halbes Äquivalents einer Diphosphorverbindung stehen kann,
- mindestens einer sterisch anspruchsvollen Stickstoffbase und
- mindestens eines Salzes
- mit Olefinen, die an der Doppelbindung mindestens ein Wasserstoff-Atom tragen
- in Gegenwart von dipolar aprotischen Lösungsmittel
umgesetzt werden.

Es sei an dieser Stelle darauf hingewiesen, dass vom Rahmen der Erfindung beliebige Kombinationen von Vorzugsbereichen mitumfasst sind.

Ar steht im Rahmen der Erfindung beispielsweise und bevorzugt für carbocyclische aromatische Reste mit 6 bis 24 Gerüstkohlenstoffatomen oder heteroaromatische Reste mit 5 bis 24 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können. Weiterhin können die carbocyclischen aromatischen Reste oder heteroaromatische Reste mit bis zu fünf gleichen oder verschiedenen Substituenten pro Cyclus substituiert sein, ausgewählt aus der Gruppe Hydroxy, Fluor, Nitro, Cyano, freies oder geschütztes Formyl, C₁-C₁₂-Alkyl, C₅-C₁₄-Aryl, C₆-C₁₅-Arylalkyl, -PO-[(C₁-C₈)-Alkyl]₂, -PO-[(C₅-C₁₄)-Aryl]₂, -PO-[(C₁-C₈)-Alkyl)(C₅-C₁₄)-Aryl)], Tri(C₁-C₈-alkyl)siloxyl oder Resten der allgemeinen Formel (II),

A-B-D-K (II)

in der unabhängig voneinander
- A: fehlt oder für einen C₁-C₈-Alkylenrest steht und
- B: fehlt oder für Sauerstoff, Schwefel oder NR¹ steht,
wobei R¹ Wasserstoff, C₁-C₉-Alkyl, C₆-C₁₅Arylalkyl oder C₅-C₁₄-Aryl bedeutet und
- D: für eine Carbonyl-Gruppe steht und
- K: für R², OR², NHR³ oder N(R³)₂ steht,
wobei R² für C₁-C₈-Alkyl, C₆-C₁₅-Arylalkyl, C₁-C₈-Halogenalkyl oder C₅-C₁₄-Aryl und
R³ jeweils unabhängig für C₁-C₈-Alkyl, C₆-C₁₅-Arylalkyl oder C₆-C₁₄-Aryl oder N(R³)₂ zusammen für einen cyclischen Aminorest steht,
oder Resten der allgemeinen Formeln (IIIa-e)

A-K (IIIa)

A-SO₂-K (IIIB)

A-B-SO₂R² (IIIc)

A-SO₃W (IIId)

A-COW (IIIe)

in denen A, B, K und R² die oben angegebene Bedeutung besitzen und W für OH, NH₂, oder OM steht, wobei M ein Alkalimetallion, ein halbes Äquivalent eines Erdalkalimetallions, ein Ammoniumion oder ein organisches Ammoniumion bedeuten kann.

Alkyl bzw. Alkylen, bzw. Alkoxy, bedeutet im Rahmen der Erfindung jeweils unabhängig einen geradkettigen, cyclischen, verzweigten oder unverzweigten Alkyl- bzw. Alkylen- bzw. Alkoxy-Rest, der gegebenenfalls durch C₁-C₄-Alkoxy-Reste weiter substituiert sein kann. Gleiches gilt für den Alkylenteil eines Arylalkyl-Restes.

Beispielsweise steht in allen Zusammenhängen bevorzugt C₁-C₆-Alkyl für Methyl, Ethyl, n-Propyl, Isopropyl, n-Butyl, tert.-Butyl, n-Pentyl, Cyclohexyl und n-Hexyl, C₁-C₈-Alkyl darüber hinaus beispielsweise für n-Heptyl, n-Octyl oder iso-Octyl, C₁-C₁₂-Alkyl, weiter darüber hinaus z.B. für n-Decyl und n-Dodecyl und C₁-C₂₀ noch weiter darüber hinaus für n-Hexadecyl und n-Octadecyl.

Beispielsweise steht C₁-C₄-Alkylen in allen Zusammenhängen bevorzugt für Methylen, 1,1-Ethylen, 1,2-Ethylen, 1,1-Propylen, 1,2-Propylen, 1,3-Propylen, 1,1-Butylen, 1,2-Butylen, 2,3-Butylen und 1,4-Butylen, C₁-C₈-Alkylen darüber hinaus für 1,5-Pentylen, 1,6-Hexylen, 1,1-Cyclohexylen, 1,4-Cyclohexylen, 1,2-Cyclohexylen und 1,8-Octylen.

Beispielsweise steht C₁-C₄-Alkoxy in allen Zusammenhängen bevorzugt für Methoxy, Ethoxy, Isopropoxy, n-Propoxy, n-Butoxy und tert.-Butoxy, C₁-C₈-Alkoxy darüber hinaus für Cyclohexyloxy.

Die allgemeine Bezeichnung Aryl als Substituent umfasst carbocyclische Reste und heteroaromatische Reste in denen keines, ein, zwei oder drei Gerüstatome pro Cyclus, im gesamten Rest mindestens jedoch ein Gerüstatom Heteroatome ausgewählt aus der Gruppe Stickstoff, Schwefel oder Sauerstoff sind. C₅-C₁₀-Aryl steht beispielsweise und bevorzugt für Phenyl, Pyridyl, o-,m-, oder p-Tolyl, C₅-C₁₄-Aryl darüber hinaus für Anthracenyl.

Gleiches gilt für den Arylteil eines Arylalkyl-Restes. C₆-C₁₅-Arylalkyl steht beispielsweise und bevorzugt für Benzyl.

Halogenalkyl bzw. Fluoralkyl bedeutet im Rahmen der Erfindung jeweils unabhängig einen geradkettigen, cyclischen, verzweigten oder unverzweigten Alkylrest, der mit einem, mehreren oder vollständig mit Halogenatomen unabhängig voneinander ausgewählt aus der Gruppe Fluor- , Chlor-, oder Brom bzw. Fluor substituiert sein können.

Beispielsweise und bevorzugt steht C₁-C₈-Halogenalkyl, in allen Zusammenhängen bevorzugt für Trifluormethyl, Trichlormethyl, 2,2,2-Trifluorethyl, Pentafluorethyl und Nonafluorbutyl, C₁-C₈-Fluoralkyl für Trifluormethyl, 2,2,2-Trifluorethyl, Pentafluorethyl und Nonafluorbutyl.

Geschütztes Formyl bedeutet einen Formyl-Rest, der durch Überführung in ein Aminal, Acetal oder ein gemischtes Aminalacetal geschützt ist, wobei die Aminale, Acetale und gemischten Aminalacetale acyclisch oder cyclisch sein können.

Beispielsweise und bevorzugt steht geschütztes Formyl für einen 1,1-(2,5-Dioxy)-cyclopentylen-Rest.

Bevorzugt werden für das erfindungsgemäße Verfahren aromatische Verbindungen der allgemeinen Formel (I) eingesetzt, in der
n = eins ist und

Ar für einen substituierten oder unsubstituierten aromatischen Rest steht und der ausgewählt ist aus der Gruppe Phenyl, Naphtyl, Phenanthrenyl, Anthracenyl, Fluorenyl, Pyridinyl, Oxazolyl, Thiophen-yl, Benzofuranyl, Benzothiophen-yl, Dibenzofuran-yl, Dibenzothiophen-yl, Furanyl, Indolyl, Pyridazinyl, Pyrazinyl, Pyrimidinyl, Triazolyl und Chinolinyl, der weiterhin mit keinem, einem, zwei oder drei Resten pro Cyclus weiter substituiert sein kann, die jeweils unabhängig voneinander ausgewählt sind aus der Gruppe Fluor, Nitro, Cyano, Di(C₁-C₆-alkyl)-amino, C₁-C₆-Alkyl, C₅-C₁₀-Aryl, C₁-C₈-Fluoralkyl, C₁-C₈-Fluoralkoxy, C₁-C₈-Alkoxy, CO(C₁-C₄-Alkyl), COO-(C₁-C₆)-Alkyl, -CON(C₁-C₆-Alkyl)₂, und
X für Chlor, Brom, Iod, Trifluormethansulfonyloxy oder Nonafluorbutansulfonyloxy steht.

Besonders bevorzugt werden für das erfindungsgemäße Verfahren aromatische Verbindungen der allgemeinen Formel (I) eingesetzt, in der
n = eins ist und
Ar für einen Phenyl-Rest steht, der mit keinem, einem, zwei oder drei Resten weiter substituiert sein kann, die jeweils voneinander unabhängig ausgewählt sind aus der Gruppe
Fluor, Cyano, C₁-C₄-Alkyl, C₁-C₄-Alkoxy, Trifluormethyl, Trifluormethoxy, Acetyl, COO-(C₁-C₆)-Alkyl, -CON(C₁-C₆-Alkyl)₂ und

X für Chlor oder Brom steht.

Ganz besonders bevorzugt werden folgende Verbindungen eingesetzt:
p-Trifluormethylchlorbenzol, o-Trifluormethylchlorbenzol, m-Trifluormethylchlorbenzol, 3,5-Bis-trifluormethylchlorbenzol, o-Cyanochlorbenzol, p-Chlorbenzaldehyd.

Als Palladiumkatalysator werden Palladiumkomplexe der Formel (IV) eingesetzt.

Monophosphorverbindungen sind beispielsweise und bevorzugt solche der allgemeinen Formel (Va)

P(E-R⁴)₃ (Va)

in der
- E: jeweils unabhängig voneinander und unabhängig von R⁴ fehlen oder für Sauerstoff stehen und die Reste R⁴ jeweils unabhängig voneinander für C₁-C₈-Alkyl oder unsubstituiertes, ein-, zwei oder dreifach durch R⁵ substituiertes Phenyl-, Naphtyl-oder Ferrocenyl stehen, wobei
R⁵ für C₁-C₈-Alkyl, C₁-C₈-Alkoxy, Chlor, Fluor-, N(C₁-C₆-Alkyl)₂, CO₂-(C₁-C₆-Alkyl), -CON(C₁-C₆-Alkyl)₂, Cyano- oder CO(C₁-C₆-Alkyl steht.

Besonders bevorzugte Monophosphorverbindungen sind solche der allgemeinen Formel (Va), in der E fehlt und R⁴ für unabhängig voneinander für C₁-C₈-Alkyl oder unsubstituiertes, ein-, zwei oder dreifach durch R⁵ substituiertes Phenyl- oder Naphtyl- oder Ferrocenyl stehen, wobei
- R⁵: für C₁-C₈-Alkyl, C₁-C₈-Alkoxy, Chlor oder Fluor steht.

Ganz besonders bevorzugt sind Monophosphorverbindungen sind solche der allgemeinen Formel (Va), in der E fehlt
und zwei oder drei der Reste R⁴ unabhängig voneinander für C₁-C₈-Alkyl und keiner oder ein Rest R⁴ für unsubstituiertes, ein-, zwei oder dreifach durch R⁵ substituiertes Phenyl- oder Naphtyl- steht, wobei
- R⁵: für C₁-C₈-Alkyl, C₁-C₈-Alkoxy, Chlor oder Fluor steht.

Noch weiter bevorzugt sind als Monophosphorverbindungen Tri-(tert.-butyl)phosphin, Phenyldi(tert.-butyl)phosphin und Ferrocenyl-di-(tert.-butyt)phosphin.

Diphosphorverbindungen können beispielsweise und bevorzugt solche der allgemeinen Formel (Vb) sein,

(R⁶-E)₂P-E-Z-E-P(E-R⁶)₂ (Vb)

in der
- E: jeweils unabhängig voneinander und unabhängig von R⁶ und Z fehlt oder für Sauerstoff steht und
die Reste R⁶ unabhängig voneinander für C₁-C₈-Alkyl oder für unsubstituiertes, ein-, zwei oder dreifach durch R⁷ substituiertes Phenyl- , Naphtyl- oder Heteroaryl mit 5 bis 12 Gerüstkohlenstoffatomen stehen, wobei
R⁷ jeweils unabhängig ausgewählt ist aus der Gruppe C₁-C₈-Alkyl, C₁-C₈-Alkoxy, Fluor- oder Cyano- und
- Z: für einen unsubstituierten oder substituierten Rest aus der Gruppe C₁-C₄-Alkylen, 1,2-Phenylen, 1,3-Phenylen, 1,2-Cyclohexyl, 1,1'-Ferrocenyl, 1,2-Ferrocenyl, 2,2'-(1,1'-Binaphtyl) und 1,1'-Biphenyl steht.

Bevorzugte Diphosphorverbindungen sind 1,3-Bis(düsopropylphosphino)propan, 1,4-Bis(diisopropylphosphino)butan, 2,2'-Bis(diphenylphosphino)-1,1'-binaphthyl.

Bevorzugt werden Komplexe eingesetzt, die als Liganden Monophosphorverbindungen enthalten.

Bevorzugte isolierte Palladiumkomplexe sind Palladium(II)bis(tri-t-butylphosphin)-dichlorid, Palladium(II)bis-di-tert.-butylphenylphosphindichlorid, Palladium(II)bis-di-tert.-butylferrocenylphosphindichlorid, Palladium(0)tricyclohexylphosphandiallylether-Komplex, Palladium(0)bistricyclohexylphosphan.

Für das erfindungsgemäße Verfahren sind als Palladiumkatalysatoren Palladiumkomplexe bevorzugt, die aus Palladium-Verbindungen und Liganden in der Reaktionslösung erzeugt werden.

Als Palladiumverbindungen können beispielsweise und bevorzugt eingesetzt werden Pd₂(di-benzylidenaceton)₃ oder Allylpalladiumchlorid oder -bromid oder solche der allgemeinen Formel (VIa),

Pd(Y¹)₂ (VIa)

in der
- Y¹: für ein Anion, bevorzugt für Chlorid, Bromid, Acetat, Propionat, Nitrat, Methansulfonat, Trifluormethansulfonat, Acetylacetonat, Allyl oder Cyclopentadienyl steht,
oder Palladiumverbindungen der allgemeinen Formel (VIb)

Pd(Y²)₂(L¹)₂ (VIb)

in der
- Y²: für ein Anion, bevorzugt Chlorid, Bromid, Acetat, Methansulfonat, Nonafluorbutansulfonat oder Trifluormethansulfonat Tetrafluoroborat oder Hexafluorophosphat steht und
- L¹: jeweils für ein Nitril, bevorzugt Acetonitril, Benzonitril oder Benzylnitril, oder ein Olefin, bevorzugt Cyclohexen oder Cycloocten, steht, oder
- (L¹)₂: zusammen für ein Diolefin, bevorzugt Norbornadien oder 1,5-Cyclooctadien steht,
oder Palladiumverbindungen der allgemeinen Formel (VIc)

M₂[Pd(Y³)₄] (VIc),

wobei
- Y³: für ein Halogenid, bevorzugt Chlorid oder Bromid steht und
- M: für Lithium, Natrium, Kalium, Ammonium oder organisches Ammonium steht.

Bevorzugt sind als Palladiumverbindungen Palladium(II)acetat, Palladium(II)chlorid, Palladium(II) bromid, Palladium(II)propionat, Palladium(11)acetylacetonat, Lithium-, Natrium- oder Kaliumtetrachloropalladat, Palladium(II)chloridbisbenzonitril, Palladium(II)chloridbisacetonitril.

Bevorzugt werden für die Erzeugung von Palladiumkomplexen in der Reaktionslösung als Liganden Phosphorverbindungen der allgemeinen Formeln (Va) und (Vb) verwendet, wobei Monophosphorverbindungen der allgemeinen Formel (Va) noch weiter bevorzugt sind. Die genannten Vorzugsbereiche gelten dabei in gleicher Weise.

Das molare Verhältnis von Phosphor zu Palladium in der Reaktionsmischung kann beispielsweise 1:1 bis 10:1 betragen, 2:1 bis 5:1 ist bevorzugt, besonders bevorzugt ist 3:1 bis 4:1.

Für das erfindungsgemäße Verfahren kann das molare Verhältnis von auszutauschendem X in Verbindungen der allgemeinen Formel (I) zu Palladium beispielsweise 10 bis 20000 betragen, bevorzugt ist ein Verhältnis von 100 bis 5000, ganz besonders bevorzugt 500 bis 2000.

Das erfindungsgemäße Verfahren wird in Gegenwart mindestens einer, bevorzugt einer, sterisch anspruchsvollen Stickstoffbase durchgeführt.

Sterisch anspruchsvolle Stickstoffbasen sind beispielsweise Amine der allgemeinen Formel

NR⁸R⁹R¹⁰ (VII)

In der R⁸, R⁹ und R¹⁰ jeweils unabhängig voneinander für C₁-C₂₀-Alkyl, C₅ bis C₁₄-Aryl oder C₆-C₁₅-Arylalkyl stehen oder jeweils zwei oder drei der Reste R⁸, R⁹ und R¹⁰ mit dem Stickstoffatom einen mono-, bi- oder tricyclischen Heterocyclus mit 4 bis 8 Kohlenstoffatomen pro Cyclus bilden kann,
wobei die Auflage gilt, dass ein, zwei oder drei der Reste R⁸, R⁹ und M¹⁰, bevorzugt zwei oder drei jeweils unabhängig voneinander entweder über ein tertiäres oder quartäres sp³-Kohlenstoffatom an das Stickstoffatom gebunden sind oder für einen Aryl-Rest stehen, der einfach oder zweifach, bevorzugt zweifach in den ortho-Positionen substituiert ist.

Reste, die über ein tertiäres oder quartäres sp³-Kohlenstoffatom an den Stickstoffatom gebunden sein können, sind beispielsweise und bevorzugt Isopropyl, sec.-Butyl, tert.-Butyl, 1-Methylbutyl, 1-Ethylpropyl, 1,1-Dimethylpropyl, 1,2-Dimethylpropyl, 1-Methylpentyl, 1,1-Dimethylbutyl, 1,2-Dimethylbutyl, 1,3-Dimethylbutyl, 1-Ethylbutyl, 1,1,2-Trimethylpropyl, 1,2,2-Trimethylpropyl, 1-Ethyl-1-methylpropyl, Cyclopentyl-, Cyclohexyl- und Cycloheptyl.

Aryl-Reste die einfach oder zweifach in den ortho-Positionen substituiert sind, sind beispielsweise o-Tolyl, 2,6-Dimethylphenyl, 2-Ethyl-6-methylphenyl, 2,6-Diisopropylphenyl, o-Anisyl und 2,6-Dimethoxyphenyl.

Monocyclische Heterocyclen im Sinne der Erfindung sind beispielsweise N-Methyl-2,2,6,6-Tetramethylpiperidin und N-Methyl-2,5-dimethylpyrolidin.

Sterisch anspruchsvolle Stickstoffbasen sind weiterhin N-heteroaromatische Verbindungen, die in beiden ortho-Positionen zum Stickstoff substituiert sind.

Bevorzugt sind das 2,6-disubstituierte Pyridine wie beispielsweise 2,6-Lutidin, 2,6-Diethylpyridin, 2,6-Diisopropylpyridin, 2,6-Dimethoxypyridin, 2,6-Di-tert.Butylpyridin.

Ganz besonders bevorzugt werden für das erfindungsgemäße Verfahren als sterisch anspruchsvolle Stickstoffbasen Ethyldiisopropylamin, Trüsopropylamin, Diisopropylanilin, Triisobutylamin, Ethyldiisobutylamin, Dicyclohexylmethylamin, Dicyclohexyethylamin, Cyclohexyldiethylamin, Cyclohexyldimethylamin und 2,6-Bis-diisopropylpyridin eingesetzt, von denen Dicyclohexylmethylamin, Dicyclohexyethylamin, Cyclohexyldimethylamin weiter bevorzugt sind.

Die Menge der eingesetzten Base kann z.B. das 0,2- bis 200-fache, bevorzugt das 1- bis 3-fache und noch weiter bevorzugt das 1,0- bis 1,2-fache, bezogen auf die molare Menge der aromatischen Verbindung der allgemeinen Formel (I) sein.

In einer Ausführungsform des erfindungsgemäßen Verfahrens kann die sterisch anspruchsvolle Stickstoffbase in Kombination mit einer anderen Base eingesetzt werden. Dazu können beispielsweise 1 bis 95 %, der Menge an sterisch anspruchsvoller Stickstoffbase durch eine nicht sterisch anspruchsvolle Stickstoffbase ersetzt werden.

Nicht sterisch anspruchsvolle Stickstoffbasen im Sinne der Erfindung sind beispielsweise Alkali- und Erdalkalimetallcarboxylate wie beispielsweise Acetate, Propionate, Benzoate, Alkali und Erdalkalimetall-Carbonate, -Hydrogencarbonate, -Phosphate, -Hydrogenphosphate, -Hydroxide. Alkalimetalle sind bevorzugt Lithium, Natrium, Kalium und Cäsium, Erdalkalimetalle bevorzugt Calcium, Magnesium und Barium.

Das erfindungsgemäße Verfahren wird in Gegenwart mindestens eines, bevorzugt eines Salzes, durchgeführt.

Als Salze sind für das erfindungsgemäße Verfahren beispielsweise und bevorzugt Salze der allgemeinen Formel (VIII) geeignet,

(Kation⁺)(Anion-) (VIII)

in der

| | |
|---|---|
| (Kation⁺) | für substituierte Ammonium, Phosphonium oder Arsonium-Kationen oder Alkalimetallionen und |
| (Anion⁻) | für das Anion einer organischen oder anorganischen Säure steht. |

Bevorzugt steht (Kation⁺) für Kationen der allgemeinen Formel (IX)

[Pnyc(C₁-C₁₂-Alkyl)ₘ(C₇-C₁₂-Arylalkyl)_{q}(C₆-C₁₀-Aryl)ᵣ]⁺ (IX)

in der
Pnyc für Stickstoff, Phosphor oder Arsen, bevorzugt für Stickstoff steht und
(m+q+r) = 4 ergibt.

Besonders bevorzugt steht (Kation⁺) für Tetrabutylammonium, Tetraphenylammonium, Tetraphenylphosphonium, Tetrabutylphosphonium.

Bevorzugt steht (Anion⁻) für Fluorid, Chlorid, Bromid, lodid, Cyanat, Thiocyanat, Acetat, Hydroxid, Nitrat, Hydrogensulfat, Tetrafluoroborat, Hexafluorophosphat, Tosylat, und Triflat, besonders bevorzugt für Chlorid, Bromid, lodid.

Ganz besonders bevorzugte Salze sind Tetrabutylammoniumchlorid, Tetrabutylammoniumbromid, Tetraphenylammoniumbromid, Tetrabutylphosphoniumchlorid, Tetrabutylphosphoniumbromid, Tetraphenylphosphoniumchlorid und Tetraphenylphosphoniumbromid oder Mischungen davon.

Noch weiter bevorzugt ist Tetrabutylammoniumbromid.

Die Salze können beispielsweise in Mengen von 0,01 - 100 mol% bezogen auf die die theoretische Ausbeute limitierende Verbindung (Arylverbindung der allgemeinen Formel (I) oder das Olefin) eingesetzt werden, bevorzugt in Mengen von 0,1 bis 15 mol%, besonders bevorzugt in Mengen von 0,5 bis 5 mol% und ganz besonders bevorzugt in Mengen von 0,5 bis 2 mol% eingesetzt werden.

Größere Mengen wie auch Salzschmelzen sind möglich, aber unwirtschaftlich.

Als Olefine, die an der Doppelbindung mindestens ein Wasserstoff-Atom tragen, können beispielsweise solche der allgemeinen Formel (X) eingesetzt werden,

R¹¹CH=CR¹²R¹³ (X)

in der unabhängig voneinander
- R¹¹: für Wasserstoff oder Methyl und
- R¹²: für Wasserstoff oder Methyl und
- R¹³: stehen kann für Wasserstoff, Cyano, SO₃M, C₁-C₈-Alkyl, carbocyclische aromatische Reste mit 6 bis 18 Gerüstkohlenstoffatomen oder heteroaromatische Reste mit 5 bis 18 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können
oder für Reste der allgemeinen Formel (XI) 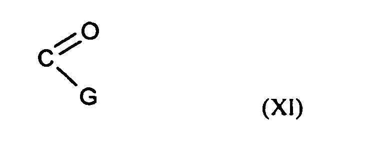 wobei
- G: für OM, OH, NH₂, OR¹⁴, NHR¹⁴ oder N(R¹⁴)₂ steht und R¹⁴ für C₁-C₁₂-Alkyl, C₆-C₁₅-Arylalkyl oder C₅-C₁₄-Aryl oder
N(R¹⁴)₂ zusammen für einen cyclischen Aminorest wie zum Beispiel Morpholino, Pyrrolidino oder Piperidino, steht und wobei M für ein Alkalimetallion, ein halbes Äquivalent eines Erdalkalimetallions, ein Ammoniumion oder ein organisches Ammoniumion stehen kann.

Die carbocyclischen aromatischen Reste und heteroaromatischen Reste können in gleicher Weise substituiert sein, wie unter den aromatischen Verbindungen der allgemeinen Formel (I) beschrieben.

Bevorzugte Beispiele für Olefine der allgemeinen Formel (X) sind Ethen, Propen, Buten, 1,1,1-Trifluor-2-propen, gegebenenfalls substituierte Vinyl-C₆-C₁₀-aromaten wie Styrol oder die isomeren Vinylnaphthaline, 2-, 3- oder 4-Fluorstyrol, 2-, 3- oder 4-Chlorstyrol, 2-, 3- oder 4-Bromstyrol, 2-, 3- oder 4-Iodstyrol, 2-, 3- oder 4-Cyanostyrol, 2-, 3- oder 4-(C₁-C₁₂)-Alkoxystyrol wie 2-, 3- oder 4-Methoxystyrol, 2-, 3- oder 4-Nitrostyrol, 2-, 3- oder 4-Styrolcarbonsäure, 2-, 3- oder 4-Styrolcarbonsäure-C₁-C₁₂-alkylester wie 2-, 3- oder 4-Styrolcarbonsäuremethylester, 2-, 3- oder 4-Styrolcarbonsäure-C₆-C₁₂-Arylester wie 2-, 3-oder 4-Styrolcarbonsäurephenylester, 2-, 3- oder 4-Styrolsulfonsäure bzw. deren Salze, 3-oder 4-Vinylphthalsäure, 3- oder 4-Vinylphthalsäuredi- C₁-C₁₂-alkylester wie 3- oder 4-Vinylphthalsäuredimethylester, 3- oder 4-Vinylphthalsäuredi-C₆-C₁₀-arylester wie 3- oder 4-Vinylphthalsäurediphenylester, 3- oder 4-Vinylphthalsäureanhydrid, Vinylhetaryle wie N-Vinylimidazol oder 2- oder 4-Vinylpyridin, ferner Acrylnitril, Acrylsäure, Acrylsäure-C₁-C₁₂-alkylester wie Acrylsäuremethylester, Acrylsäureethylester, Acrylsäure-n-propylester, Acrylsäure-2-ethyl-hexylester, Acrylsäureamid, Vinylsulfonsäure und deren Sulfonate.

Als Olefine mit mindestens einem Wasserstoff-Substituenten ganz besonders bevorzugt sind Ethylen, Propen, Acrylnitril, Acrylsäure, Acrylsäuremethylester, Acrylsäure(2-ethylhexyl)ester, Acrylsäureamid, 1,1,1-Trifluor-2-propen und Styrol, wobei Acrylnitril, Acrylsäuremethylester, Acrylsäureamid und Styrol noch weiter bevorzugt sind.

Die Menge des eingesetzten Olefins kann beispielsweise das 0,2 bis 200-fache (bei Verwendung als Lösungsmittel) bezogen auf die molare Menge der aromatischen Verbindung der allgemeinen Formel (I) sein, das 0,5- bis 5-fache ist bevorzugt, das 0,8- bis 1,2-fache ist ganz besonders bevorzugt. Noch weiter bevorzugt ist das 0,9- bis 1,0-fache.

Werden aromatische Verbindungen der allgemeinen Formel (I) oder Olefine der allgemeinen Formel (X) verwendet, die freie Säuregruppe wie z.B. Sulfonsäure- oder Carbonsäuregruppen tragen, so ist die Menge der eingesetzten Base, sterisch anspruchsvollen Stickstoffbase oder der nicht sterisch anspruchsvollen Stickstoffbase entsprechend zu erhöhen.

Das erfindungsgemäße Verfahren wird in Gegenwart von dipolar aprotischem Lösungsmittel durchgeführt.

Bevorzugte dipolar aprotische Lösungsmittel sind

Ether wie z.B. Dioxan, THF, 1,2-Dimethoxyethan, Diethylenglykoldimethylether, Tetraethylenglykoldimethylether.

Amidische Lösungsmittel wie beispielsweise Dimethylformamid, N-Methylpyrrolidon, N-Methylcaprolactam oder Dimethylacetamid.

Sulfoxide und Sulfone wie zum Beispiel Dimethylsulfoxid oder Tetramethylensulfon oder Mischungen solcher Lösungsmittel.

Nitrile wie z.B. Acetonitril, Benzonitril und Benzylnitril, Ketone wie z.B. Dimethylketon, Diethylketon, Methyl-tert.-butylketon.

Die Menge des gegebenenfalls eingesetzten Lösungsmittels kann beispielsweise 50 ml bis 5000 ml bevorzugt 100 bis 500 ml pro Mol der aromatischen Verbindung der allgemeinen Formel (I) sein.

Die Reaktionstemperatur kann zum Beispiel 20°C bis 200°C, bevorzugt 80 bis 150°C und besonders bevorzugt 100°C bis 140 °C betragen.

Die Reaktion kann beispielsweise bei 0,2 bis 100 bar durchgeführt werden, bevorzugt ist Normaldruck.

Die Reaktionsdauer kann beispielsweise 0,2 h bis 72 Stunden betragen, 1 bis 10 h sind bevorzugt.

Die Reaktion wird bevorzugt unter Schutzgasatmosphäre unter weitgehendem Ausschluss von Sauerstoff und Feuchtigkeit durchgeführt. Als Schutzgase kommen beispielsweise Stickstoff und Edelgase wie beispielsweise Argon oder Mischungen solcher Gase in Frage.

In einer bevorzugten Ausführungsform des erfindungsgemäßen Verfahrens legt man in einem Reaktionsgefäß die aromatische Verbindung der allgemeinen Formel (I) zusammen mit dem Olefin, der Base, dem Salz, dem Liganden, und der Palladiumverbindung in einem Reaktionsgefäß unter Schutzgas vor und erwärmt den Ansatz unter Rühren auf die Reaktionstemperatur. Nach beendeter Reaktion gießt man die Mischung auf Wasser. Feste Produkte fallen dann aus und können abgesaugt und z.B. mit Wasser gewaschen werden. Flüssige Produkte können mit einem organischen, mit Wasser nicht oder wenig mischbaren Lösungsmittel extrahiert und beispielsweise destillativ aufgearbeitet werden.

Feste Produkte können gegebenenfalls z.B. durch Umkristallisieren oder Umfällen weiter gereinigt werden.

Es kann von Vorteil sein, die Reaktion dosierkontrolliert durchzuführen, indem man im Lauf der Reaktion das Olefin bei Reaktionstemperatur zudosiert.

Es kann von Vorteil sein, gewisse Mengen eines Radikalinhibitor, wie z.B. 2,6-Di-tert.-butylphenol zuzusetzen um radikalische Nebenreaktionen zu vermeiden.

Alternativ kann auch der Palladiumkatalysator erst im Verlauf der Reaktion zugegeben werden oder durch Zugabe von Ligand oder Palladiumverbindung im Lauf der Reaktion erzeugt werden. Auch die simultane Zudosierung von Olefinen und Palladiumkatalysator bzw. Ligand bzw. Palladiumverbindung ist möglich.

Es ist von Vorteil, bei der Aufarbeitung eine schwach saure wässrige Lösung zu verwenden um gegebenenfalls verbleibende Base als Salz zu binden. Die Base kann beispielsweise durch Alkalisieren und Extrahieren der Waschflüssigkeit mit einem organischen Lösungsmittel zurückgewonnen werden.

Auf erfindungsgemäße Weise werden Arylolefine der allgemeinen Formel (XII) erhalten

Ar-(R¹¹C=R¹²R¹³)ₙ (XII)

in der
- Ar und n: die unter der allgemeinen Formel (I) und R¹¹, R¹², R¹³ die unter der allgemeinen Formel (X) genannte Bedeutung besitzen.

Das erfindungsgemäße Verfahren eignet sich insbesondere für die Herstellung von Arylacrylsäurederivaten der allgemeinen Formel (XIII)

Ar-(R¹¹ = R¹²R¹³) (XIII)

in der
- Ar: die unter der allgemeinen Formel (I) und R¹¹, R¹² die unter der allgemeinen Formel (X) angegebene Bedeutung besitzt und R¹³ für Cyano oder Reste der allgemeinen Formel (XI) mit der dort genannten Bedeutung steht.

Der Vorteil des erfindungsgemäßen Verfahrens liegt in der leichten Durchführbarkeit und den hohen Ausbeuten an aromatischen Olefinen. Weiterhin werden hohe Katalysatorumsatzzahlen (TON) von über 100 Mol Halogenaromat/Mol Palladium-Katalysator sowie hohe Katalysatorumsatzraten von über 50 pro Stunde (TOF) erreicht.

### Beispiele

### Beispiel 1

### Synthese von 3-Trifluormethylzimtamid (Methode I)

In einem Schlenkgefäß werden 0,512 g (7,2 mmol) Acrylamid, 38,7 mg (120 µmol) NBu₄Br, 6,7 mg (30 µmol) Palladiumacetat und 26,7 mg (120 µmol) Phenyldi(t-butyl)phosphan eingewogen. Dann werden 1,08 g (6 mmol) 3-Chlorbenzotrifluorid und 1,53 ml (7,2 mmol) Dicyclohexylmethylamin sowie 3,5 ml Dimethylacetamid zugegeben. Das Schlenkgefäß wird in ein Heizbad von 120°C gestellt und der Inhalt gerührt. Nach 4 h wird der Inhalt auf 30 ml Wasser gegossen und das Produkt mit t-Butylmethylether ausgeschüttelt. Nach Trocknen der organischen Phase über MgSO₄ wird das Lösungsmittel im Vakuum entfernt und das Produkt isoliert. Gelbliches Öl, Ausbeute: 917 mg (71 % d. Th.)

### Beispiel 2

### Synthese von 3-Trifluormethylzimtamid (Methode II)

In einem Schlenkgefäß werden 0,181 g (2,55 mmol) Acrylamid, 9,7 mg (30 µmol) NBu₄Br, 0,675 mg (3 µmol) Palladiumacetat und 2,67 mg (12 µmol) Phenyldi(t-butyl)phosphan eingewogen. Dann werden 0,542 g (3 mmol) 3-Chlorbenzotrifluorid und 0,637 ml (3 mmol) Dicyclohexylmethylamin sowie 2,5 ml Dimethylacetamid zugegeben. Das Schlenkgefäß wird in ein Heizbad von 130°C gestellt und der Inhalt gerührt. Nach 3 h wird die Ausbeute über HPLC bestimmt: 64 % (TON 544, TOF 181 /h).

### Beispiel 3

### Synthese von 4-Trifluormethylzimtamid (Methode (I)

In einem Schlenkgefäß werden 0,512 g (7,2 mmol) Acrylamid, 23,2 mg (72 µmol) NBu₄Br, 3,2 mg (14,4 µmol) Palladiumacetat und 12,8 mg (58 µmol) Phenyldi(t-butyl)phosphan eingewogen. Dann werden 1,56 g (8,63 mmol) 4-Chlorbenzotrifluorid und 1,83 ml (8,63 mmol) Dicyclohexylmethylamin sowie 4 ml Dimethylacetamid zugegeben. Das Schlenkgefäß wird in ein Heizbad von 110°C gestellt und der Inhalt gerührt. Nach 24 h wird der Inhalt auf 30 ml Wasser gegossen, das feste Produkt abgesaugt und mit ca. 50 ml Wasser gewaschen. Farbloser Feststoff, Ausbeute nach dem Trocknen 1,47 g (94,8 % d. Th.).

### Beispiel 4

### Synthese von 4-Trifluormethylzimtamid (Methode (II)

Wie Beipiel 6, jedoch wurden nur 1,6 mg (7,2 µmol) Palladiumacetat und 6,4 mg (29 µmol) Phenyldi(t-butyl)phosphan eingesetzt. Dabei wurde die Reaktionstemperatur auf 120°C erhöht und bereits nach nur 4 Stunden eine Ausbeute von 1,36 g (88% d.Th.; TON 880; TOF 220 /h) erhalten.

### Beispiel 5

### Synthese von 4-Trifluormethylzimtamid (Methode (III)

In einem Schlenkgefäß werden 0,178 g (2,5 mmol) Acrylamid, 10 mg (30 µmol) NBu₄Br, 1,2 mg (6 µmol) Palladiumacetat und 4,8 mg (24 µmol) Phenyldi(t-butyl)phosphan eingewogen. Dann werden 0,541 g (3 mmol) 4-Chlorbenzotrifluorid und 0,754 g (3,6 mmol) Dicyclohexylethylamin sowie 2 ml Dimethylacetamid zugegeben. Das Schlenkgefäß wird in ein Heizbad von 130°C gestellt und der Inhalt gerührt. Nach 4 h werden 200 µl der Reaktionslösung entnommen und mit 3,8 ml Methyl-tert.-butylether verdünnt. Dann wird die Probe mit HPLC untersucht. Ausbeute: 100 %, zum gewünschten Produkt.

### Beispiel 6

### Synthese von 4-Trifluormethylzimtamid (Methode (IV)

In einem Schlenkgefäß werden 0,178 g (2,5 mmol) Acrylamid, 10 mg (30 µmol) NBu₄Br, 1,2 mg (6 µmol) Palladiumacetat und 4,8 mg (24 µmol) Phenyldi(t-butyl)phosphan eingewogen. Dann werden 0,541 g (3 mmol) 4-Chlorbenzotrifluorid und 0,559 g (3,6 mmol) Cyclohexyldiethylamin sowie 2 ml Dimethylacetamid zugegeben. Das Schlenkgefäß wird in ein Heizbad von 130°C gestellt und der Inhalt gerührt. Nach 4 h werden 200 µl der Reaktionslösung entnommen und mit 3,8 ml Methyl-tert.-butylether verdünnt. Dann wird die Probe mit HPLC untersucht. Ausbeute: 100 %, zum gewünschten Produkt.

### Beispiel 7

### Synthese von 4-Trifluormethylzimtamid (Methode (V)

In einem Schlenkgefäß werden 0,178 g (2,5 mmol) Acrylamid, 10 mg (30 µmol) NBu₄Br, 1,2 mg (6 µmol) Palladiumacetat und 4,8 mg (24 µmol) Phenyldi(t-butyl)phosphan eingewogen. Dann werden 0,541 g (3 mmol) 4-Chlorbenzotrifluorid und 0,458 g (3,6 mmol) Cyclohexyldimethylamin sowie 2 ml Dimethylacetamid zugegeben. Das Schlenkgefäß wird in ein Heizbad von 130°C gestellt und der Inhalt gerührt. Nach 4 h werden 200 µl der Reaktionslösung entnommen und mit 3,8 ml Methyl-tert.-butylether verdünnt. Dann wird die Probe mit HPLC untersucht. Ausbeute: 100 %, zum gewünschten Produkt.

### Beispiel 8

### Synthese von 4-Trifluormethylzimtamid (Methode (VI)

In einem Schlenkgefäß werden 0,178 g (2,5 mmol) Acrylamid, 10 mg (30 µmol) NBu₄Br, 1,2 mg (6 µmol) Palladiumacetat und 4,8 mg (24 µmol) Phenyldi(t-butyl)phosphan eingewogen. Dann werden 0,541 g (3 mmol) 4-Chlorbenzotrifluorid und 0,465 g (3,6 mmol) Ethyldüsopropylamin sowie 2 ml Dimethylacetamid zugegeben. Das Schlenkgefäß wird in ein Heizbad von 130°C gestellt und der Inhalt gerührt. Nach 4 h werden 200 µl der Reaktionslösung entnommen und mit 3,8 ml Methyl-tert.-butylether verdünnt. Dann wird die Probe mit HPLC untersucht. Ausbeute: 96 %, zum gewünschten Produkt.

### Beispiel 9

### Synthese von 3,5-Bis(trifluormethyl)zimtamid (Methode I)

In einem Schlenkgefäß werden 0,512 g (7,2 mmol) Acrylamid, 38,7 mg (120 µmol) NBu₄Br, 6,7 mg (30 µmol) Palladiumacetat und 26,7 mg (120 µmol) Phenyldi(t-butyl)phosphan eingewogen. Dann werden 1,49 g (6 mmol) 3,5-Bis(trifluormethyl)chlorbenzol und 1,53 ml (7,2 mmol) Dicyclohexylmethylamin sowie 3,5 ml Dimethylacetamid zugegeben. Das Schlenkgefäß wird in ein Heizbad von 130°C gestellt und der Inhalt gerührt. Nach 4 h wird der Inhalt auf 30 ml Wasser gegossen, das feste Produkt abgesaugt und mit ca. 50 ml Wasser gewaschen. Hellgrauer Feststoff, Ausbeute nach dem Trocknen 1,63 g (96 % d. Th.).

### Beispiel 10

### Synthese von 3,5-Bis(trifluormethyl)zimtamid (Methode II)

In einem Schlenkgefäß werden 0,181 g (2,55 mmol) Acrylamid, 9,7 mg (30 µmol) NBu₄Br, 0,675 mg (3 µmol) Palladiumacetat und 2,67 mg (12 µmol) Phenyldi(t-butyl)phosphan eingewogen. Dann werden 0,542 g (3 mmol) 3,5-Bis(trifluormethyl)-chlorbenzol und 0,637 ml (3 mmol) Dicyclohexylmethylamin sowie 2,5 ml Dimethylacetamid zugegeben. Das Schlenkgefäß wird in ein Heizbad von 130°C gestellt und der Inhalt gerührt. Nach 3 h wird die Ausbeute über HPLC bestimmt: 99 % (TON 723, TOF 241 /h).

### Beispiel 11

### Synthese von 3-Cyano-4-trifluormethylzimtamid

In einem Schlenkgefäß werden 0,512 g (7,2 mmol) Acrylamid, 38,7 mg (120 µmol) NBu₄Br, 6,7 mg (30 µmol) Palladiumacetat und 26,7 mg (120 µmol) Phenyldi(t-butyl)phosphan eingewogen. Dann werden 1,23 g (6 mmol) 3-Cyano-4-trifluormethylchlorbenzol und 1,53 ml (7,2 mmol) Dicyclohexylmethylamin sowie 3,5 ml Dimethylacetamid zugegeben. Das Schlenkgefäß wird in ein Heizbad von 130°C gestellt und der Inhalt gerührt. Nach 4 h wird der Inhalt auf 30 ml Wasser gegossen, das feste Produkt abgesaugt und mit ca. 50 ml Wasser gewaschen. Hellgrauer Feststoff, Ausbeute nach dem Trocknen 1,26 g (87 % d. Th.).

### Beispiel 12

### Synthese von 3-Methyl-4-trifluormethylzimtamid

In einem Schlenkgefäß werden 0,512 g (7,2 mmol) Acrylamid, 38,7 mg (120 µmol) NBu₄Br, 6,7 mg (30 µmol) Palladiumacetat und 26,7 mg (120 µmol) Phenyldi(t-butyl)phosphan eingewogen. Dann werden 1,17 g (6 mmol) 3-Methyl-4-trifluormethylchlorbenzol und 1,53 ml (7,2 mmol) Dicyclohexylmethylamin sowie 3,5 ml Dimethylacetamid zugegeben. Das Schlenkgefäß wird in ein Heizbad von 130°C gestellt und der Inhalt gerührt. Nach 4 h wird der Inhalt auf 30 ml Wasser gegossen, das feste Produkt abgesaugt und mit ca. 50 ml Wasser gewaschen. Hellgrauer Feststoff, Ausbeute nach dem Trocknen 1,29 g (92,8 % d. Th.).

### Beispiel 13

### Synthese von 3,5-Bis(trifluormethyl)zimtsäuremethylester

In einem Schlenkgefäß werden 0,619 g (7,2 mmol) Acrylsäuremethylester, 38,7 mg (120 µmol) NBu₄Br, 6,7 mg (30 µmol) Palladiumacetat und 26,7 mg (120 µmol) Phenyldi(t-butyl)phosphan eingewogen. Dann werden 1,49 g (6 mmol) 3,5-Bis(trifluormethyl)-chlorbenzol und 1,53 ml (7,2 mmol) Dicyclohexylmethylamin sowie 3,5 ml Dimethylacetamid zugegeben. Das Schlenkgefäß wird in ein Heizbad von 130°C gestellt und der Inhalt gerührt. Nach 4 h wird der Inhalt auf 30 ml Wasser gegossen und das Produkt mit t-Butylmethylether ausgeschüttelt. Nach Trocknen der organischen Phase über MgSO₄ wird das Lösungsmittel im Vakuum entfernt und das Produkt isoliert. Gelblicher, kristalliner Feststoff, Ausbeute: 1,34 g (97 % d. Th.).

### Beispiel 14

### Synthese von 3-Cyano-4-trifluormethylzimtsäuremethylester

In einem Schlenkgefäß werden 0,619 g (7,2 mmol) Acrylsäuremethylester, 38,7 mg (120 µmol) NBu₄Br, 6,7 mg (30 µmol) Palladiumacetat und 26,7 mg (120 µmol) Phenyldi(t-butyl)phosphan eingewogen. Dann werden 1,23 g (6 mmol) 3-Cyano-4-trifluormethylchlorbenzol und 1,53 ml (7,2 mmol) Dicyclohexylmethylamin sowie 3,5 ml Dimethylacetamid zugegeben. Das Schlenkgefäß wird in ein Heizbad von 130°C gestellt und der Inhalt gerührt. Nach 4 h wird der Inhalt auf 30 ml Wasser gegossen und das Produkt mit t-Butylmethylether ausgeschüttelt. Nach Trocknen der organischen Phase über MgSO₄ wird das Lösungsmittel im Vakuum entfernt und das Produkt isoliert. Gelblicher, kristalliner Feststoff, Ausbeute: 1,31 g (85,5 % d. Th.).

### Beispiel 15

### Synthese von 3-Methyl-4-trifluormethylzimtsäuremethylester

In einem Schlenkgefäß werden 0,619 g (7,2 mmol) Acrylsäuremethylester, 38,7 mg (120 µmol) NBu₄Br, 6,7 mg (30 µmol) Palladiumacetat und 26,7 mg (120 µmol) Phenyldi(t-butyl)phosphan eingewogen. Dann werden 1,17 g (6 mmol) 3-Methyl-4-trifluormethylchlorbenzol und 1,53 ml (7,2 mmol) Dicyclohexylmethylamin sowie 3,5 ml Dimethylacetamid zugegeben. Das Schlenkgefäß wird in ein Heizbad von 130°C gestellt und der Inhalt gerührt. Nach 4 h wird der Inhalt auf 30 ml Wasser gegossen und das Produkt mit t-Butylmethylether ausgeschüttelt. Nach Trocknen der organischen Phase über MgSO₄ wird das Lösungsmittel im Vakuum entfernt und das Produkt isoliert. Gelblicher, kristalliner Feststoff. Ausbeute: 86 %, zwei Produkte im Verhältnis 99:1 (trans/cis-Produkt).

### Beispiel 16

### Synthese von 3,5-Bis(trifluormethyl)zimtsäurenitril

In einem Schlenkgefäß werden 0,382 g (7,2 mmol) Acrylonitril, 38,7 mg (120 µmol) NBu₄Br, 6,7 mg (30 µmol) Palladiumacetat und 26,7 mg (120 µmol) Phenyldi(t-butyl)phosphan eingewogen. Dann werden 1,49 g (6 mmol) 3,5-Bis(trifluormethyl)-chlorbenzol und 1,53 ml (7,2 mmol) Dicyclohexylmethylamin sowie 3,5 ml Dimethylacetamid zugegeben. Das Schlenkgefäß wird in ein Heizbad von 130°C gestellt und der Inhalt gerührt. Nach 4 h wird der Inhalt auf 30 ml Wasser gegossen und das Produkt mit t-Butylmethylether ausgeschüttelt. Nach Trocknen der organischen Phase über MgSO₄ wird das Lösungsmittel im Vakuum entfernt und das Produkt isoliert. Oranges Öl, Ausbeute: 69%.

### Beispiel 17

### Synthese von 3-Trifluormethyl-trans-stilben

In einem Schlenkgefäß werden 0,745 g (7,2 mmol) Styrol, 38,7 mg (120 µmol) NBu₄Br, 6,7 mg (30 µmol) Palladiumacetat und 26,7 mg (120 µmol) Phenyldi(t-butyl)phosphan eingewogen. Dann werden 1,08 g (6 mmol) 3-Chlorbenzotrifluorid und 1,53 ml (7,2 mmol) Dicyclohexylmethylamin sowie 3,5 ml Dimethylacetamid zugegeben. Das Schlenkgefäß wird in ein Heizbad von 130°C gestellt und der Inhalt gerührt. Nach 4 h wird der Inhalt auf 30 ml Wasser gegossen und das Produkt mit t-Butylmethylether ausgeschüttelt. Nach Trocknen der organischen Phase über MgSO₄ wird das Lösungsmittel im Vakuum entfernt und das Produkt isoliert. Gelblicher, kristalliner Feststoff, Ausbeute: 1,43 g (96 % d. Th.)

### Beispiel 18

### Synthese von 4-Methoxyphenylzimtamid

In einem Schlenkgefäß werden 0,181 g (2,55 mmol) Acrylamid, 9,7 mg (30 µmol) NBu₄Br, 0,068 mg (0,3 µmol) Palladiumacetat und 0,27 mg (1,2 µmol) Phenyldi(t-butyl)phosphan eingewogen. Dann werden 0,561 g (3 mmol) 4-Bromanisol und 0,637 ml (3 mmol) Dicyclohexylmethylamin sowie 2,5 ml Dimethylacetamid zugegeben. Das Schlenkgefäß wird in ein Heizbad von 130°C gestellt und der Inhalt gerührt. Nach 3 h wird die Ausbeute über HPLC bestimmt: 100 % Ausbeute (TON 8500, TOF 2833 /h); zwei isomere Produkte im Verhältnis 61 / 39.

## Patentansprüche

1. Verfahren zur Herstellung von Arylolefinen, **dadurch gekennzeichnet, dass** aromatische Verbindungen der allgemeinen Formel (I),
Ar-[X]ₙ (I),
in der
n für eins oder zwei steht und
Ar für einen substituierten oder unsubstituierten aromatischen Rest und
X jeweils unabhängig für Chlor, Brom, Iod oder ein Sulfonat steht
in Gegenwart eines Palladium-Katalysators der allgemeinen Formel (IV)
[PdL₂An₂] (IV)
in der
L für jeweils eine Monophosphorverbindung oder
L₂ zusammen für ein Diphosphorverbindung und
An für ein Anion, bevorzugt für Chlorid, Bromid, Iodid, Acetat, Propionat, Allyl oder Cyclopentadienyl steht.
oder solche der allgemeinen Formel (IVb)
[PdLₙ] (IVb)
in der n für 2, 3 oder 4 und
in der
L jeweils für eine Monophosphorverbindung oder ein halbes Äquivalents einer Diphosphorverbindung stehen kann,
- mindestens einer sterisch anspruchsvollen Stickstoffbase und
- mindestens eines Salzes
- mit Olefinen, die an der Doppelbindung mindestens ein Wasserstoff-Atom tragen
in Gegenwart von dipolar aprotischem Lösungsmittel umgesetzt werden.

2. Verfahren nach Anspruch 1, **dadurch gekennzeichnet, dass** es in Gegenwart von Lösungsmittel durchgeführt wird.

3. Verfahren nach einem oder mehreren der Ansprüche 1 bis 2, **dadurch gekennzeichnet, dass** in der allgemeinen Formel (I)
Ar steht für carbocyclische aromatische Reste mit 6 bis 24 Gerüstkohlen-stoffatomen oder heteroaromatische Reste mit 5 bis 24 Gerüstkohlenstoff-atomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sind, wobei
die carbocyclischen aromatischen Reste oder heteroaromatische Reste mit bis zu fünf gleichen oder verschiedenen Substituenten pro Cyclus substituiert sind, ausgewählt aus der Gruppe Hydroxy, Fluor, Nitro, Cyano, freies oder geschütztes Formyl, C₁-C₁₂-Alkyl, C₅-C₁₄-Aryl, C₆-C₁₅-Arylalk, -PO-[(C₁-C₈)-Alkyl]₂, -PO-[(C₅-C₁₄)-Aryl]₂, -PO-[(C₁-C₈)-Alkyl)(C₅-C₁₄)-Aryl)], Tri(C₁-C₈-alkyl)siloxyl oder Resten der allgemeinen Formel (II),
A-B-D-K (II)
in der unabhängig voneinander
A fehlt oder für einen C₁-C₈-Alkylenrest steht und
B fehlt oder für Sauerstoff, Schwefel oder NR¹ steht,
wobei R¹ Wasserstoff, C₁-C₈-Alkyl, C₆-C₁₅-Arylalkyl oder C₅-C₁₄-Aryl bedeutet und
D für eine Carbonyl-Gruppe steht und
K für R², OR², NHR³ oder N(R³)₂ steht,
wobei R² für C₁-C₈-Allcyl, C₆-C₁₅-Arylalkyl, C₁₋C₈-Halogenalkyl oder C₅-C₁₄-Aryl und
R³ jeweils unabhängig für C₁-C₈-Alkyl, C₆-C₅-Arylalkyl oder C₆-C₁₄-Aryl- oder N(R³)₂ zusammen für einen cyclischen Aminorest steht,
oder Resten der allgemeinen Formeln (IIIa-e)
A-K (IIIa)
A-SO₂- (IIIb)
A-B-SO₂R² (IIIc)
A-SO₃W (IIId)
A-COW (IIIe)
in denen
A, B, K und R² die oben angegebene Bedeutung besitzen und W für OH, NH₂, oder OM steht, wobei M ein Alkalimetallion, ein halbes Äquivalent eines Erdalkalimetallions, ein Ammoniumion oder ein organisches Ammoniumion bedeutet und
X für Chlor, Brom, Iod, Trifluormethansulfonyloxy oder Nonafluorbutan-sulfonyloxy steht.

4. Verfahren nach einem oder mehreren der Ansprüche 1 bis 3, **dadurch gekennzeichnet, dass** als aromatische Verbindungen solche der allgemeinen Formel (I) eingesetzt werden, in der
n = eins ist und
Ar für einen substituierten oder unsubstituierten aromatischen Rest steht, ausgewählt aus der Gruppe Phenyl, Naphtyl, Phenanthrenyl, Anthracenyl, Fluorenyl, Pyridinyl, Oxazolyl, Thiophen-yl, Benzofuranyl, Benzothiophen-yl, Dibenzofuran-yl, Dibenzothiophen-yl, Furanyl, Indolyl, Pyridazinyl, Pyrazinyl, Pyrimidinyl, Triazolyl und Chinolinyl, und der weiterhin mit keinem, einem, zwei oder drei Resten pro Cyclus weiter substituiert sein kann, die jeweils unabhängig voneinander ausgewählt sind aus der Gruppe Fluor, Nitro, Cyano, Di(C₁-C₆-alkyl)-amino, C₁-C₆-Alkyl, C₆-C₁₄-Aryl, C₁-C₈-Fluoralkyl, G₁-C₈-Fluoralkoxy, C₁-C₈-Alkoxyl CO(C₁-C₄-Alkyl), COO-(C₁-C₄)-Alkyl, -CON(C₁-C₆-Alkyl)₂, und
X für Chlor, Brom, Iod, Trifluormethansulfonyloxy oder Nonafluorbutan-sulfonyloxy steht.

5. Verfahren nach einem oder mehreren der Ansprüche 1 bis 4, **dadurch gekennzeichnet, dass** in der allgemeinen Formel (I)
X für Chlor steht.

6. Verfahren nach einem oder mehreren der Ansprüche 1 bis 5, **dadurch gekennzeichnet, dass** als Palladiumkatalysatoren Palladiumkomplexe eingesetzt werden.

7. Verfahren nach einem oder mehreren der Ansprüche 1 bis 6, **dadurch gekennzeichnet, dass** als Palladiunlkatalysatoren Palladiumkomplexe eingesetzt werden, die aus Palladium-Verbindungen und Phosphorverbindungen in der Reaktionslösung erzeugt werden.

8. Verfahren nach Anspruch 7, **dadurch gekennzeichnet, dass** als Phosphorverbindungen Monophosphorverbindungen der allgemeinen Formel (Va) eingesetzt werden,
P(E-R⁴)₃ (Va)
in der
E jeweils unabhängig voneinander und unabhängig von R⁴ fehlen oder für Sauerstoff stehen und
die Reste R⁴ jeweils unabhängig voneinander für C₁-C₈-Alkyl oder unsubstituiertes, ein-, zwei oder dreifach durch R⁵ substituiertes Phenyl-, Naphtyl- oder Ferrocenyl stehen, wobei
R⁵ für C₁-C₈-Alkyl, C₁-C₈-Alkoxy, Chlor, Fluor-, N(C₁-C₆-Alkyl)₂, CO₂-(C₁-C₆-Alkyl), -CON(C₁-C₆-Alkyl)₂, Cyano- oder CO(C₁-C₆-Alkyl) steht oder
Diphosphorverbindungen der allgemeinen Formel (Vb) eingesetzt werden,
(R⁶-E)₂P-E-Z-E-P(E-R⁶)₂ (Vb)
in der
E jeweils unabhängig voneinander und unabhängig von R⁶ und Z fehlt oder für Sauerstoff steht und
die Reste R⁶ unabhängig voneinander für C₁-C₈-Alkyl oder für unsubstituiertes, ein-, zwei oder dreifach durch R⁷ substituiertes Phenyl-, Naphtyl-oder Heteroaryl mit 5 bis 12 Gerüstkohlenstoffatomen stehen, wobei
R⁷ jeweils unabhängig ausgewählt ist aus der Gruppe C₁-C₈-Alkyl, C₁-C₈-Alkoxy, Fluor- oder Cyano- und
Z für einen unsubstituierten oder substituierten Rest aus der Gruppe C₁-C₄-Alkylen, 1,2-Phenylen, 1,3-Phenylen, 1,2-Cyclohexylen, 1,1'-Ferrocenylen, 1,2-Ferrocenylen, 2,2'-(1,1'-Binaphtylen) und 1,1'-Biphenylen steht

9. Verfahren nach einem oder mehreren der Ansprüche 7 bis 8, **dadurch gekennzeichnet, dass** als Phosphorverbindungen Tri-(tert.-butyl)phosphin, Phenyldi(tert.-butyl)phosphin und Ferrocenyl-di-(tert.-butyl)phosphin verwendet werden.

10. Verfahren nach einem oder mehreren der Ansprüche 7 bis 9, **dadurch gekennzeichnet, dass** das molare Verhältnis von Phosphor zu Palladium in der Reaktionsmischung 1:1 bis 10:1 beträgt.

11. Verfahren nach einem oder mehreren der Ansprüche 7 bis 10, **dadurch gekennzeichnet, dass** das molare Verhältnis von Phosphor zu Palladium in der Reaktionsmischung 3:1 bis 4:1 beträgt.

12. Verfahren nach einem oder mehreren der Ansprüche 1 bis 11, **dadurch gekennzeichnet, dass** das molare Verhältnis von X in Verbindungen der allgemeinen Formel (I) zu Palladium 10 bis 20000 beträgt.

13. Verfahren nach einem oder mehreren der Ansprüche 1 bis 12, **dadurch gekennzeichnet, dass** als sterisch anspruchsvolle Stickstoffbasen Amine der allgemeinen Formel eingesetzt werden,
NR⁸R⁹R¹⁰ (VII)
in der
R⁸, R⁹ und R¹⁰ jeweils unabhängig voneinander für C₁-C₂₀-Alkyl, C₅- bis C₁₄-Aryl oder C₆-C₁₅-Arylalkyl stehen oder jeweils zwei oder drei der Reste R⁸, R⁹ und R¹⁰ mit- dem Stickstoffatom einen mono-, bi- oder tricyclischen Heterocyclus mit 4 bis 8 Kohlenstoffatomen pro Cyclus bilden kann,
wobei die Auflage gilt, dass ein, zwei oder drei der Reste R⁸, R⁹ und R¹⁰ jeweils unabhängig voneinander entweder über ein tertiäres oder quartäres sp³-Kohlenstoffatom an das Stickstoffatom gebunden sind oder für einen Aryl-Rest stehen, der einfach oder zweifach, in den ortho-Positionen substituiert ist oder
N-heteroaromatische Verbindungen, die in beiden ortho-Positionen zum Stickstoff substituiert sind.

14. Verfahren nach einem oder mehreren der Ansprüche 1 bis 13, **dadurch gekennzeichnet, dass** als sterisch anspruchsvolle Stickstoffbasen Ethyldiisopropylamin, Triisopropylamin, Diisopropylanilin, Triisobutylamin, Ethyldiisobutylamin, Dicycloheylmethylamin, Dicyclohexyethylamin, Cyclohexyldiethylamin, Cyclohexyldimethylamin und Bis-diisopropylpyridin eingesetzt werden.

15. Verfahren nach einem oder mehreren der Ansprüche 1 bis 14, **dadurch gekennzeichnet, dass** als Salze solche der allgemeinen Formel (VIII) eingesetzt werden,
(Kation⁺)(Anion⁻) (VIII)
in der
| | |
|---|---|
| (Kation⁺) | für substituierte Ammonium, Phosphonium, Arsoniumkationen oder Alkalimetallionen steht, und |
| (Anion⁻) | für das Anion einer organischen oder anorganischen Säure steht. |

16. Verfahren nach einem oder mehreren der Ansprüche 1 bis 15, **dadurch gekennzeichnet, dass** als Salze Tetrabutylammoniumchlorid, Tetrabutylammoniumbromid, Tetraphenylammoniumbromid, Tetrabutylphosphoniumchlorid, Tetrabutylphosphoniumbromid, Tetraphenylphosphoniumchlorid und Tetraphenylphosphoniumbromid oder Mischungen davon eingesetzt werden..

17. Verfahren nach einem oder mehreren der Ansprüche 1 bis 16, **dadurch gekennzeichnet, dass** die Salze in Mengen von 0,5 - 2 mol-% bezogen auf die die theoretische Ausbeute limitierende Verbindung eingesetzt werden.

18. Verfahren nach einem oder mehreren der Ansprüche 1 bis 17, **dadurch gekennzeichnet, dass** als Olefine, die an der Doppelbindung mindestens ein Wasserstoff Atom tragen solche der allgemeinen Formel (X) eingesetzt werden,
R¹¹CH=CR¹²R¹³ (X)
in der unabhängig voneinander
R¹¹ für Wasserstoff oder Methyl und
R¹² für Wasserstoff oder Methyl und
R¹³ steht für Wasserstoff, Cyano, SO₃M, C₁-C₈-Alkyl, carbocyclische aromatische Reste mit 6 bis 18 Gerüstkohlenstoffatomen oder hetero-aromatische Reste mit 5 bis 18 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können oder für Reste der allgemeinen Formel (XI) 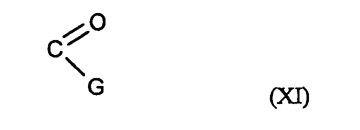
wobei
G für OM, OH, NH₂, OR¹⁴, NHR¹⁴ oder N(R¹⁴)₂ steht und R¹⁴ für C₁-C₁₂-Alkyl, C₆-C₁₅-Arylalkyl oder C₆-C₁₀-Aryl oder
N(R¹⁴)₂ zusammen für einen cyclischen Aminorest steht und wobei M für ein Alkalimetallion, ein halbes Äquivalent eines Erdalkalimetallions, ein Ammoniumion oder ein organisches Ammoniumion steht.

19. Verfahren nach einem oder mehreren der Ansprüche 1 bis 18, **dadurch gekennzeichnet, dass** als Olefine mit mindestens einem Wasserstoff-Substituenten Ethylen, Propen, Acrylnitril, Acrylsäure, Acrylsäuremethylester, Acrylsäure(2-ethylhexyl)ester, Acrylsäureamid, 1,1,1-Trifluor-2-propen und Styrol eingesetzt werden.

20. Verfahren nach einem oder mehreren der Ansprüche 1 bis 19, **dadurch gekennzeichnet, dass** als Olefine mit mindestens einem Wasserstoff-Substituenten Acrylnitril, Acrylsäuremethylester, Acrylsäureamid und Styrol eingesetzt werden.

21. Verfahren nach einem oder mehreren der Ansprüche 1 bis 20, **dadurch gekennzeichnet, dass** die Reaktionstemperatur 20°C bis 200°C beträgt.

## Claims

1. Process for preparing arylolefins, **characterized in that**
- aromatic compounds of the general formula I),
Ar-[X]ₙ (I),
where
n is one or two and
Ar is a substituted or unsubstituted aromatic radical and
X are each, independently of one another, chlorine, bromine, iodine or a sulphonate,
- are reacted in the presence of a dipolar aprotic solvent with olefins which bear at least one hydrogen atom on the double bond
- in the presence of a palladium catalyst of the general formula (IV),
[PdL₂An₂] (IV)
where
L is in each case a monophosphorus compound or
L₂ together represents a diphosphorus compound and
An is an anion, preferably chloride, bromide, iodide, acetate, propionate, allyl or cyclopentadienyl,
or of the general formula (IVb)
[PdLₙ] (IVb)
where n is 2, 3 or 4 and
L is in each case a monophosphorus compound or can represent half an equivalent of a diphosphorus compound,
- at least one bulky nitrogen base and
- at least one salt.

2. Process according to Claim 1, **characterized in that** it is carried out in the presence of a solvent.

3. Process according to Claim 1 or 2, **characterized in that**, in the general formula (I),
Ar is a carbocyclic aromatic radical having from 6 to 24 framework carbon atoms or a heteroaromatic radical having from 5 to 24 framework carbon atoms in which no, one, two or three framework carbon atoms per ring, but at least one framework carbon atom in the total molecule, is/are replaced by heteroatoms selected from the group consisting of nitrogen, sulphur and oxygen, where
the carbocyclic aromatic radical or heteroaromatic radical is substituted by up to five identical or different substituents per ring selected from the group consisting of hydroxy, fluoro, nitro, cyano, free or protected formyl, C₁-C₁₂-alkyl, C₅-C₁₄-aryl, C₆-C₁₅-arylalkyl, -PO-[(C₁-C₈)-alkyl]₂, -PO- [(C₅-C₁₄) -aryl]₂, -PO-[(C₁-C₈) -alkyl) (C₅-C₁₄) -aryl)], tri (C₁-C₈-alkyl)siloxyl and radicals of the general formula (II),
A-B-D-K (II)
where, independently of one another,
A is absent or is a C₁-C₈-alkylene radical and
B is absent or is oxygen, sulphur or NR¹,
where R¹ is hydrogen, C₁-C₈-alkyl, C₆-C₁₅-arylalkyl or C₅-C₁₄-aryl and
D is a carbonyl group and
K is R², OR², NHR³ or N(R³)₂,
where R² is C₁-C₈-alkyl, C₆-C₁₅-arylalkyl, C₁-C₈-haloalkyl or C₅-C₁₄-aryl and
R³ are each, independently of one another, C₁-C₈-alkyl, C₆-C₁₅-arylalkyl or C₆-C₁₄-aryl or the moiety N(R³)₂ is a cyclic amino radical,
and radicals of the general formulae (IIIa-e)
A-K (IIIa)
A-SO₂-K (IIIb)
A-B-SO₂R² (IIIc)
A-SO₃W (IIId)
A-COW (IIIe)
where
A, B, K and R² are as defined above and W is OH, NH₂, or OM, where M is an alkali metal ion, half an equivalent of an alkaline earth metal ion, an ammonium ion or an organic ammonium ion and
X is chlorine, bromine, iodine, trifluoromethanesulphonyloxy or nonafluorobutanesulphonyloxy.

4. Process according to one or more of Claims 1 to 3, **characterized in that** aromatic compounds used are compounds of the general formula (I) in which
n = one and
Ar is a substituted or unsubstituted aromatic radical selected from the group consisting of phenyl, naphthyl, phenanthrenyl, anthracenyl, fluorenyl, pyridinyl, oxazolyl, thiophenyl, benzofuranyl, benzothiophenyl, dibenzofuranyl, dibenzothiophenyl, furanyl, indolyl, pyridazinyl, pyrazinyl, pyrimidinyl, triazolyl and quinolinyl and may also be further substituted by no, one, two or three radicals per ring which are selected independently from the group consisting of fluorine, nitro, cyano, di(C₁-C₆-alkyl)amino, C₁-C₆-alkyl, C₆-C₁₄-aryl, C₁-C₈-fluoroalkyl, C₁-C₈-fluoroalkoxy, C₁-C₈-alkoxy, CO(C₁-C₄-alkyl), COO-(C₁-C₄)-alkyl, -CON(C₁-C₆-alkyl)₂, and
X is chlorine, bromine, iodine, trifluoromethanesulphonyloxy or nonafluorobutanesulphonyloxy.

5. Process according to one or more of Claims 1 to 4, **characterized in that**, in the general formula (I),
X is chlorine.

6. Process according to one or more of Claims 1 to 5, **characterized in that** the palladium catalysts used are palladium complexes.

7. Process according to one or more of Claims 1 to 6, **characterized in that** the palladium catalysts used are palladium complexes which are generated in the reaction solution from palladium compounds and phosphorus compounds.

8. Process according to Claim 7, **characterized in that** the phosphorus compounds used are monophosphorus compounds of the general formula (Va),
P(E-R⁴)₃ (Va)
where
E are each, independently of one another and independently of R⁴, absent or oxygen and the radicals R⁴ are each, independently of one another, C₁-C₈-alkyl or unsubstituted phenyl, naphthyl or ferrocenyl or phenyl, naphthyl or ferrocenyl substituted by one, two or three radicals R⁵, where
R⁵ is C₁-C₈-alkyl, C₁-C₈-alkoxy, chlorine, fluorine, N(C₁-C₆-alkyl)₂, CO₂-(C₁-C₆-alkyl), -CON(C₁-C₆-alkyl)₂, cyano or CO (C₁-C₆-alkyl) or
diphosphorus compounds of the general formula (Vb),
(R⁶-E)₂P-E-Z-E-P (E-R⁶)₂ (Vb)
where
E are each, independently of one another and independently of R⁶ and Z, absent or oxygen and
the radicals R⁶ are each, independently of one another, C₁-C₈-alkyl or phenyl, naphthyl or heteroaryl having from 5 to 12 framework carbon atoms which may be unsubstituted or substituted by one, two or three radicals R⁷, where
R⁷ are selected independently from the group consisting of C₁-C₈-alkyl, C₁-C₈-alkoxy, fluorine and cyano and
Z is an unsubstituted or substituted radical selected from the group consisting of C₁-C₄-alkylene, 1,2-phenylene, 1,3-phenylene, 1,2-cyclohexylene, 1,1'-ferrocenylene, 1,2-ferrocenylene, 2,2'-(1,1'-binaphthylene) and 1,1'-biphenylene.

9. Process according to Claim 7 or 8, **characterized in that** phosphorus compounds used are tri(tert-butyl)phosphine, phenyldi(tert-butyl)phosphine and ferrocenyldi(tert-butyl)phosphine.

10. Process according to one or more of Claims 7 to 9 **characterized in that** the molar ratio of phosphorus to palladium in the reaction mixture is from 1:1 to 10:1.

11. Process according to one or more of Claims 7 to 10, **characterized in that** the molar ratio of phosphorus to palladium in the reaction mixture is from 3:1 to 4:1.

12. Process according to one or more of Claims 1 to 11, **characterized in that** the molar ratio of X in compounds of the general formula (I) to palladium is from 10 to 20 000.

13. Process according to one or more of Claims 1 to 12, **characterized in that** the bulky nitrogen bases used are amines of the general formula,
NR⁸R⁹R¹⁰ (VII)
where
R⁸ R⁹ and R¹⁰ are each, independently of one another, C₁-C₂₀-alkyl, C₅-C₁₄-aryl or C₆-C₁₅-arylalkyl or two or three of the radicals R⁸, R⁹ and R¹⁰ together with the nitrogen atom may form a monocyclic, bicyclic or tricyclic heterocycle having from 4 to 8 carbon atoms per ring,
with the proviso that one, two or three of the radicals R⁸, R⁹ and R¹⁰ are each, independently of one another, either bound to the nitrogen atom via a tertiary or quaternary sp³ carbon atom or are an aryl radical which is monosubstituted or disubstituted in the ortho positions or
N-heteroaromatic compounds which are substituted in the two ortho positions relative to the nitrogen.

14. Process according to one or more of Claims 1 to 13, **characterized in that** bulky nitrogen bases used are ethyldiisopropylamine, triisopropylamine, diisopropylaniline, triisobutylamine, ethyldiisobutylamine, dicyclohexylmethylamine, dicyclohexylethylamine, cyclohexyldiethylamine, cyclohexyldimethylamine and bis(diisopropyl)pyridine.

15. Process according to one or more of Claims 1 to 14, **characterized in that** salts used are ones of the general formula (VIII),
(Cation⁺) (Anion⁻) (VIII)
where
| | |
|---|---|
| (Cation⁺) | is a substituted ammonium, phosphonium or arsonium cation or an alkali metal ion, and |
| (Anion⁻) | is the anion of an organic or inorganic acid. |

16. Process according to one or more of Claims 1 to 15, **characterized in that** salts used are tetrabutylammonium chloride, tetrabutylammonium bromide, tetraphenylammonium bromide, tetrabutylphosphonium chloride, tetrabutylphosphonium bromide, tetraphenylphosphonium chloride and tetraphenylphosphonium bromide or mixtures thereof.

17. Process according to one or more of Claims 1 to 16, **characterized in that** the salts are used in amounts of 0.5-2 mol% based on the theoretical yield-limiting compound.

18. Process according to one or more of Claims 1 to 17, **characterized in that** the olefins bearing at least one hydrogen atom on the double bond are olefins of the general formula (X),
R¹¹CH=CR¹²R¹³ (X)
where, independently of one another,
R¹¹ is hydrogen or methyl and
R¹² is hydrogen or methyl and
R¹³ is hydrogen, cyano, SO₃M, C₁-C₈-alkyl, a carbocyclic aromatic radical having from 6 to 18 framework carbon atoms or a heteroaromatic radical having from 5 to 18 framework carbon atoms in which no, one, two or three framework carbon atoms per ring, but at least one framework carbon atom in the total molecule, may be replaced by heteroatoms selected from the group consisting of nitrogen, sulphur and oxygen or a radical of the general formula (XI) 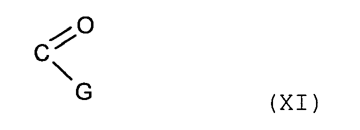
where
G is OM, OH, NH₂, OR¹⁴, NHR¹⁴ or N(R¹⁴)₂, and R¹⁴ is C₁-C₁₂-alkyl, C₆-C₁₅-arylalkyl or C₆-C₁₀-aryl or
the moiety N(R¹⁴)₂ is a cyclic amino radical and M is an alkali metal ion, half an equivalent of an alkaline earth metal ion, an ammonium ion or an organic ammonium ion.

19. Process according to one or more of Claims 1 to 18, **characterized in that** the olefins having at least one hydrogen substituent which are used are ethylene, propene, acrylonitrile, acrylic acid, methyl acrylate, 2-ethylhexyl acrylate, acrylamide, 1,1,1-trifluoro-2-propene and styrene.

20. Process according to one or more of Claims 1 to 19, **characterized in that** the olefins having at least one hydrogen substituent which are used are acrylonitrile, methyl acrylate, acrylamide and styrene.

21. Process according to one or more of Claims 1 to 20, **characterized in that** the reaction temperature is from 20°C to 200°C.

## Revendications

1. Procédé pour la préparation d'aryloléfines, **caractérisé en ce qu'**on transforme
- des composés aromatiques de formule générale (I)
Ar-[X]ₙ (I)
dans laquelle
n vaut un ou deux et
Ar représente un radical aromatique substitué ou non substitué et
X représente à chaque fois, indépendamment, chlore, brome, iode ou sulfonate
- en présence d'un catalyseur de palladium de formule générale (IV)
[PdL₂An₂] (IV),
dans laquelle
L représente à chaque fois un composé monophosphoré ou
L₂ représente ensemble un composé diphosphoré et
An représente un anion, de préférence chlorure, bromure, iodure, acétate, propionate, allyle ou cyclopentadiényle
ou de formule générale (IVb)
[PdLₙ] (IVb)
dans laquelle n vaut 2, 3 ou 4 et
dans laquelle
L peut représenter à chaque fois un composé monophosphoré ou un demi-équivalent d'un composé diphosphoré,
- au moins une base azotée stériquement encombrée et
- au moins un sel
- avec des oléfines qui portent au moins un atome d'hydrogène sur la double liaison
en présence d'un solvant aprotique dipolaire.

2. Procédé selon la revendication 1, **caractérisé en ce qu'**il est réalisé en présence de solvants.

3. Procédé selon l'une ou plusieurs des revendications 1 à 2, **caractérisé en ce que**, dans la formule générale (I)
Ar représente des radicaux aromatiques carbocycliques comprenant 6 à 24 atomes de carbone de structure ou des radicaux hétéroaromatiques comprenant 5 à 24 atomes de carbone de structure, dans lesquels aucun, un, deux ou trois atomes de carbone de structure par cycle, dans la totalité de la molécule cependant au moins un atome de carbone de structure est substitué par des hétéroatomes, choisis dans le groupe formé par azote, soufre ou oxygène
les radicaux aromatiques ou hétéroaromatiques carbocycliques étant substitués par jusqu'à cinq substituants identiques ou différents par cycle, choisis dans le groupe formé par hydroxy, fluor, nitro, cyano, formyle libre ou protégé, C₁-C₁₂-alkyle, C₅-C₁₄-aryle, C₆-C₁₅-arylalkyle, -PO-[(C₁-C₈) -alkyle]₂, -PO- [(C₈-C₁₄)-aryle)₂, -PO- [(C₁-C₈)-alkyl) (C₅-C₁₄) -aryle)], tri(C₁-C₈-alkyl) siloxyle ou des radicaux de formule générale (II),
A-B-D-K (II)
dans laquelle, indépendamment l'un de l'autre
A manque ou représente un radical C₁-C₈-alkylène et
B manque ou représente oxygène, soufre ou NR¹, où R¹ signifie hydrogène, C₁-C₈-alkyle, C₆-C₁₅-arylalkyle ou C₅-C₁₄-aryle et
D représente un groupe carbonyle et
K représente R², OR², NHR³ ou N(R³)₂,
où R² représente C₁-C₈-alkyle, C₆-C₁₅-arylalkyle, C₁-C₈-halogénoalkyle ou C₅-C₁₄-aryle et
R³ représente, à chaque fois indépendamment, C₁-C₈-alkyle, C₆-C₁₅-arylalkyle ou C₆-C₁₄-aryle ou N(R³)₂ représente ensemble un radical amino cyclique,
ou des radicaux des formules générales (IIIa-e)
A-K (IIIa)
A-SO₂-K (IIIb)
A-B-SO₂R² (IIIc)
A-SO₃W (IIId)
A-COW (IIIe)
dans lesquelles
A, B, K et R² présentent la signification susmentionnée et W représente OH, NH₂ ou OM, où M signifie un ion de métal alcalin, un demi-équivalent d'un ion de métal alcalino-terreux, un ion d'ammonium ou un ion d'ammonium organique et
X représente chlore, brome, iode, trifluorométhanesulfonyloxy ou nonafluorobutanesulfonyloxy.

4. Procédé selon l'une ou plusieurs des revendications 1 à 3, **caractérisé en ce qu'**on utilise comme composés aromatiques ceux de formule générale (I), dans laquelle
n = un et
Ar représente un radical substitué ou non substitué, aromatique, choisi dans le groupe formé par phényle, naphtyle, phénanthrényle, anthracényle, fluorényle, pyridinyle, oxazolyle, thiophényle, benzofurannyle, benzothiophényle, dibenzofurannyle, dibenzothiophényle, furannyle, indolyle, pyridazinyle, pyrazinyle, pyrimidinyle, triazolyle et quinoléinyle, et qui peut en outre être substitué par aucun, un, deux ou trois radicaux par cycle, qui sont à chaque fois choisis, indépendamment l'un de l'autre, dans le groupe formé par fluor, nitro, cyano, di (C₁-C₆-alkyl) -amino, C₁-C₆-alkyle, C₆-C₁₄-aryle, C₁-C₈-fluoroalkyle, C₁-C₈-fluoroalcoxy, C₁-C₈-alcoxy, CO(C₁-C₄-alkyle), COO-(C₁-C₄) -alkyle, -CON(C₁-C₆-alkyle)₂, et
X représente chlore, brome, iode, trifluorométhanesulfonyloxy ou nonafluorobutanesulfonyloxy.

5. Procédé selon l'une ou plusieurs des revendications 1 à 4, **caractérisé en ce que**, dans la formule générale (I)
X représente chlore.

6. Procédé selon l'une ou plusieurs des revendications 1 à 5, **caractérisé en ce qu'**on utilise, comme catalyseurs de palladium des complexes de palladium.

7. Procédé selon l'une ou plusieurs des revendications 1 à 6, **caractérisé en ce qu'**on utilise comme catalyseurs de palladium des complexes de palladium, qui sont produits à partir de composés de palladium et de composés de phosphore dans la solution réactionnelle.

8. Procédé selon la revendication 7, **caractérisé en ce qu'**on utilise comme composés du phosphore des composés monophosphorés de formule générale (Va),
P(E-R⁴)₃ (Va)
dans laquelle
E à chaque fois indépendamment l'un de l'autre et indépendamment de R⁴, manque ou représente oxygène et
les radicaux R⁴ représentent, indépendamment l'un de l'autre, C₁-C₈-alkyle ou phényle, naphtyle ou ferrocényle non substitué, monosubstitué, disubstitué ou trisubstitué par R⁵, où
R⁵ représente C₁-C₈-alkyle, C₁-C₈-alcoxy, chlore, fluor, N(C₁-C₆-alkyle)₂, CO₂- (C₁-C₆-alkyle), -CON (C₁-C₆-alkyle) ₂, cyano ou CO (C₁-C₆-alkyle) ou
des composés diphosphorés de formule générale (Vb),
(R⁶-E)₂P-E-Z-E-P(E-R⁶)2 (Vb)
dans laquelle
E à chaque fois indépendamment l'un de l'autre et indépendamment de R⁶ et de Z, manque ou représente oxygène et
les radicaux R⁶ représentent, indépendamment l'un de l'autre, C₁-C₈-alkyle ou phényle, naphtyle ou hétéroaryle comprenant 5 à 12 atomes de carbone de structure, non substitué, monosubstitué, disubstitué ou trisubstitué par R⁷, où
R⁷ est choisi, à chaque fois indépendamment, dans le groupe formé par C₁-C₈-alkyle, C₁-C₈-alcoxy, fluor ou cyano et
Z représente un radical non substitué ou substitué du groupe formé par C₁-C₄-alkylène, 1,2-phénylène, 1,3-phénylène, 1,2-cyclohexylène, 1,1'-ferrocénylène, 1,2-ferrocénylène, 2,2'-(1,1'-binaphtylène) et 1,1'-biphénylène.

9. Procédé selon l'une ou plusieurs des revendications 7 à 8, **caractérisé en ce qu'**on utilise comme composés phosphorés la tri-(tert-butyl)phosphine, la phényldi(tert-butyl)phosphine et la ferrocényldi-(tert-butyl)phosphine.

10. Procédé selon l'une ou plusieurs des revendications 7 à 9, **caractérisé en ce que** le rapport molaire de phosphore à palladium dans le mélange réactionnel est de 1:1 à 10:1.

11. Procédé selon l'une ou plusieurs des revendications 7 à 10, **caractérisé en ce que** le rapport molaire de phosphore à palladium dans le mélange réactionnel est de 3:1 à 4:1.

12. Procédé selon l'une ou plusieurs des revendications 1 à 11, **caractérisé en ce que** le rapport molaire de X dans les composés de formule générale (I) à palladium est de 10 à 20 000.

13. Procédé selon l'une ou plusieurs des revendications 1 à 12, **caractérisé en ce qu'**on utilise comme bases azotées stériquement encombrées des amines de formule générale
NR⁸R⁹R¹⁰ (VII)
dans laquelle
R⁸, R⁹ et R¹⁰ représentent, à chaque fois indépendamment l'un de l'autre, C₁-C₂₀-alkyle, C₅-C₁₄-aryle ou C₆-C₁₅-arylalkyle ou à chaque fois deux ou trois des radicaux R⁸, R⁹ et R¹⁰ peuvent former avec l'atome d'azote un hétérocycle monocyclique, bicyclique ou tricyclique comprenant 4 à 8 atomes de carbone par cycle,
à condition qu'un, deux ou trois des radicaux R⁸, R⁹ et R¹⁰, à chaque fois indépendamment l'un de l'autre, soient liés à l'atome d'azote via un atome de carbone tertiaire ou quaternaire sp³ ou représentent un radical aryle, qui est monosubstitué ou disubstitué dans les positions ortho ou
des composés N-hétéroaromatiques, qui sont substitués dans les deux positions ortho par rapport à l'azote.

14. Procédé selon l'une ou plusieurs des revendications 1 à 13, **caractérisé en ce qu'**on utilise, comme bases azotées stériquement encombrées l'éthyldiisopropylamine, la triisopropylamine, la diisopropylaniline, la triisobutylamine, l'éthyldiisobutylamine, la dicyclohexylméthylamine, la dicyclohexyléthylamine, la cyclohexyldiéthylamine, la cyclohexyldiméthylamine et la bisdiisopropylpyridine.

15. Procédé selon l'une ou plusieurs des revendications 1 à 14, **caractérisé en ce qu'**on utilise comme sels ceux de formule générale (VIII),
(cation⁺) (anion⁻) (VIII)
dans laquelle
| | |
|---|---|
| (cation⁺) | représente des cations ammonium, phosphonium, arsénium substitués ou des ions de métal alcalin, et |
| (anion⁻) | représente l'anion d'un acide organique ou inorganique. |

16. Procédé selon l'une ou plusieurs des revendications 1 à 15, **caractérisé en ce qu'**on utilise, comme sels, le chlorure de tétrabutylammonium, le bromure de tétrabutylammonium, le bromure de tétraphénylammonium, le chlorure de tétrabutylphosphonium, le bromure de tétrabutylphosphonium, le chlorure de tétraphénylphosphonium et le bromure de tétraphénylphosphonium ou les mélanges de ceux-ci.

17. Procédé selon l'une ou plusieurs des revendications 1 à 16, **caractérisé en ce que** les sels sont utilisés en des quantités de 0,5-2% en mole par rapport au composé limitant le rendement théorique.

18. Procédé selon l'une ou plusieurs des revendications 1 à 17, **caractérisé en ce qu'**on utilise comme oléfines, qui portent au moins un atome d'hydrogène sur la double liaison, celles de formule générale (X),
R¹¹CH=CR¹²R¹³ (X)
dans laquelle, indépendamment l'un de l'autre
R¹¹ représente hydrogène ou méthyle et
R¹² représente hydrogène ou méthyle et
R¹³ représente hydrogène, cyano, SO₃M, C₁-C₈-alkyle, des radicaux aromatiques carbocycliques comprenant 6 à 18 atomes de carbone de structure ou des radicaux hétéroaromatiques comprenant 5 à 18 atomes de carbone de structure, dans lesquels aucun, un, deux ou trois atomes de carbone de structure par cycle, dans la totalité de la molécule cependant au moins un atome de carbone de structure peut être substitué par des hétéroatomes, choisis dans le groupe formé par azote, soufre ou oxygène ou représente des radicaux de formule générale (XI) 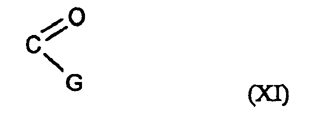
où
G représente OM, OH, NH₂, OR¹⁴, NHR¹⁴ ou N(R¹⁴)₂ et R¹⁴ représente C₁-C₁₂-alkyle, C₆-C₁₅-arylalkyle ou C₆-C₁₀-aryle ou
N(R¹⁴)₂ représente ensemble un radical amino cyclique et où M représente un ion de métal alcalin, un demi-équivalent d'un ion de métal alcalino-terreux, un ion d'ammonium ou un ion d'ammonium organique.

19. Procédé selon l'une ou plusieurs des revendications 1 à 18, **caractérisé en ce qu'**on utilise comme oléfines avec au moins un substituant hydrogène, l'éthylène, le propylène, l'acrylonitrile, l'acide acrylique, l'ester méthylique de l'acide acrylique, l'ester 2-éthylhexylique de l'acide acrylique, l'amide de l'acide acrylique, le 1,1,1-trifluoro-2-propylène et le styrène.

20. Procédé selon l'une ou plusieurs des revendications 1 à 19, **caractérisé en ce qu'**on utilise comme oléfines avec au moins un substituant hydrogène, l'acrylonitrile, l'ester méthylique de l'acide acrylique, l'amide de l'acide acrylique et le styrène.

21. Procédé selon l'une ou plusieurs des revendications 1 à 20, **caractérisé en ce que** la température de réaction est de 20°C à 200°C.
